# EUROPÄISCHE PATENTSCHRIFT

(11) **EP 1 066 406 B1**
(45) Veröffentlichungstag und Bekanntmachung des Hinweises auf die Patenterteilung: **25.08.2004**
(21) Anmeldenummer: 99916843.8
(22) Anmeldetag: 22.03.1999
(51) Int. Cl.: C12Q 1/37, C12Q 1/26, C12Q 1/32

(54) **BESTIMMUNG VON PROTEINASEN MIT HILFE EINES ENZYMS ALS PROTEINASESUBSTRAT**
DETECTION OF PROTEINASES BY MEANS OF AN ENZYME AS PROTEINASE SUBSTRATE
DETECTION DE PROTEINASES AU MOYEN D'UNE ENZYME COMME SUBSTRAT DE PROTEINASE

(30) Priorität: 02.04.1998 DE 19814761
(43) Veröffentlichungstag der Anmeldung: 10.01.2001
(73) Patentinhaber: MERCK PATENT GmbH, 64293 Darmstadt (DE)
(72) Erfinder: LINXWEILER, Winfried, 64823 Gross-Umstadt (DE)
(86) Internationale Anmeldenummer: PCT/EP1999/001874
(87) Internationale Veröffentlichungsnummer: WO 1999/051768

(56) Entgegenhaltungen:
- EP-A- 0 810 290
- FELICIOLI R; ROMAGNOLI A; GIACHI A; BERNINI S; BALESTRERI E: "THE USE OF A NATIVE ENZYME PROTEIN AS SUBSTRATE FOR PLANT PROTEINASE ASSAY" JOURNAL OF PLANT PHYSIOLOGY, Bd. 132, Nr. 1, 1988, Seiten 34-37, XP002114481 in der Anmeldung erwähnt
- NJUS D; BALDWIN T O; HASTINGS J W: "A sensitive assay for proteolytic enzymes using bacterial luciferase as a substrate" ANALYTICAL BIOCHEMISTRY, Bd. 61, Nr. 1, September 1974 (1974-09), Seiten 280-287, XP002114482
- DERGOUSOVA N I; AMERIK AYU; VOLYNSKAYA A M; RUMSH L D: "HIV-I protease. Cloning, expression, and purification" APPLIED BIOCHEMISTRY AND BIOTECHNOLOGY, Bd. 61, Nr. 1-2, Oktober 1996 (1996-10) - November 1996 (1996-11), Seiten 97-107, XP002114483
- NAGGIE, S.; HU, Y. C.; PULLIAM-HOLOMAN, T. R.; BENTLEY, W. E.: "Substrate (gelatin) gel electrophoretic method for analysis of protease activity in insect (Sf-9) cells" BIOTECHNOLOGY TECHNIQUES, Bd. 11, Nr. 5, 1997, Seiten 297-300, XP002114484
- ORSTAN A; GAFNI A: "Inhibition of proteinase K by phosphorylated sugars" BIOCHEMISTRY INTERNATIONAL, Bd. 25, Nr. 4, November 1991 (1991-11), Seiten 657-662, XP002114485
- BERREZ, JEAN MARC; LATRUFFE, NORBERT; GAUDEMER, YVES: "Limited proteolysis of D-.beta.-hydroxybutyrate dehydrogenase: relationships between phospholipid-protein interactions and catalytical activity" BIOCHEMISTRY INTERNATIONAL, Bd. 8, Nr. 5, 1984, Seiten 697-706, XP002114486

## Beschreibung

Die Erfindung betrifft Verfahren und Mittel zur Bestimmung von Proteinasen in biologischen Flüssigkeiten und in Lebensmitteln mit Hilfe eines Enzyms, das als hochempfindliches Substrat für Proteinasen dient.

Proteinasen sind in allen lebenden Zellen und Geweben vorhanden. Beim Aufschluß von tierischen und pflanzlichen Zellen oder Zellen von Mikroorganismen werden diese Proteinasen freigesetzt. Sie stören bei der Isolierung oder Analytik von Proteinen, weil sie zu deren Abbau führen können. Deshalb müssen die Proteinasen abgetrennt, inaktiviert oder inhibiert werden. Um dies zu tun, ist eine empfindliche Bestimmung der Proteinaseaktivität wichtig.

Proteinasen in Lebensmitteln stammen entweder aus dem Lebensmittel selbst oder von Mikroorganismen, welche die Lebensmittel kontaminieren. Im letzteren Fall kann der Nachweis von Proteinasen als Hinweis auf bakterielle Kontamination dienen. Weiterhin sind Proteinasen oft die Ursache von Lebensmittelverderb, vor allem, wenn die Enzyme durch die Methoden der Entkeimung der Lebensmittel nicht vollständig inaktiviert werden. Solche Proteinasen werden z.B. durch Bakterien der Gattungen Bacillus oder Pseudomonas gebildet. Es ist z.B. bekannt, daß die sogenannte Süßgerinnung von ultrahocherhitzter Milch auf die Wirkung von Proteinasen aus Bakterien wie *Pseudomonas fluorescens* zurückzuführen ist.

Proteolytische Enzyme, insbesondere Endoproteinasen, in biologischen Flüssigkeiten wie Urin oder Ausscheidungen wie Faeces sind z.B. PMN-Elastase, die aus Granulozyten stammt, oder Trypsin und Chymotrypsin aus dem Pankreas. Eine Änderung der Konzentration dieser Endoproteinasen kann Krankheitszustände oder bestimmte physiologische Zustände anzeigen. Die schnelle und empfindliche Bestimmung der Konzentration ist für die Diagnose dieser Zustände wichtig.

Für die Charakterisierung von Mikroorganismen ist die Bestimmung ihrer proteolytischen Aktivität wichtig. So werden z.B. Clostridien nach ihrer Fähigkeit Proteinasen zu bilden charakterisiert. Vor allem Bakterien der Gattung Pseudomonas bilden Proteinasen und scheiden sie aus.

Verfahren zur Bestimmung von Proteinasen sind aus dem Stand der Technik bekannt (Proteolytic Enzymes - A Practical Approach, edited by R J Beynon & J S Bond, IRL Press at Oxford University Press, Oxford New York Tokyo, "Protease assay methods" G. Sarath et al. 25-55, 1989). Als spezifische Substrate für Proteinasen dienen dabei natürliche Proteine wie Casein, Hämoglobin, Kollagen oder modifizierte Proteine wie z.B. Azocasein. Auch synthetische Peptide, die chromophore oder fluorophore Gruppen (z.B. 4-nitroanilid bzw. 7-amino-4-methylcumarin) tragen, werden als Substrate für die Bestimmung von Proteinasen eingesetzt. Diese Substrate sind jedoch sehr spezifisch für bestimmte Proteinasen und deshalb nicht geeignet, als ein generelles Substrat für proteolytische Enzyme verwendet zu werden.
Weiterhin können radioaktiv markierte Proteine oder Peptide als Proteinasesubstrate verwendet werden, um die Sensitivität des Tests zu erhöhen. Die bekannten Nachteile bei der Verwendung radioaktiv markierter Substanzen schließen diese Methoden für eine breitere Anwendung im Lebensmittelbereich und der Routineanalytik aus.

Die Nachteile dieser Verfahren sind die begrenzte Empfindlichkeit, insbesondere bei Proben mit hohem Proteingehalt wie Lebensmitteln oder Zellaufschlüssen und die Störungen durch gefärbte oder trübe Proben. Die notwendige Trennung des nichtverdauten Substrats von den proteolytischen Fragmenten durch Säurefällung oder die Verwendung einer festen Phase sind zusätzliche Arbeitsschritte, die möglichst vermieden werden sollten. Weiter ist bei den empfindlicheren Methoden mit Fluoreszenzmessung ein hoher apparativer Aufwand und eine Probenvorbereitung notwendig.

Es besteht daher ein dringender Bedarf für einen empfindlichen Test auf Proteinasen, der mit wenigen Arbeitsschritten ausgeführt wird und der photochemisch, reflektometrisch oder visuell ausgeführt werden kann. Störungen durch trübe oder gefärbte Proben oder durch Proben mit hohem Proteingehalt, wie sie bei biologischen Proben und Lebensmitteln vorliegen, sollen weitgehend vermieden werden.

Eine Möglichkeit, die Trennung von nicht abgebautem Substrat von den proteolytischen Fragmenten zu vermeiden, ist die Verwendung von Enzymen als Substrat. Proteinasen können Enzyme inaktivieren. Durch die empfindliche Messung der Reaktivität kann dann die Aktivität der Proteinase gemessen werden.
Aus SU 1067441 ist eine Proteinasebestimmung bekannt, bei der das Enzym Luciferase als Proteinasesubstrat verwendet wird. Über einen Zeitraum von 7-13 Stunden wird der Abfall der Luciferaseaktivität gemessen und aus der Inaktivierungskonstanten wird die Proteinaseaktivität berechnet. Die Empfindlichkeit dieser Methode ist mit 5 ng/ml Trypsin im Testansatz angegeben.

In J. Plant Physiol. 132, 34 (1988) wird ein Verfahren beschrieben, bei dem das Enzym Aldolase als Substrat für eine Proteinasebestimmung eingesetzt wird. Die proteolytische Aktivität wird durch den Abfall der Aldolaseaktivität gemessen.

Nachteile dieser Testmethoden mit Enzymen als Substrat sind die langen Inkubationszeit und die begrenzte Nachweisempfindlichkeit.
Die Aufgabenstellung war daher, einen Test zu entwickeln, dessen Zeitbedarf 5 Stunden nicht überschreitet und dessen Nachweisempfindlichkeit gegenüber den im Stand der Technik genannten Methoden deutlich verbessert ist.

Überraschenderweise wurde gefunden, daß viele inaktivierte Enzyme verbesserte Eigenschaften als Proteinasesubstrat aufweisen, und daß anschließend nach dem proteolytischen Abbau und nach der Reaktivierung des Enzyms die Empfindlichkeit eines Proteinasetests im Vergleich zur Verwendung des nicht inaktivierten Enzyms mindestens um den Faktor 20 gesteigert werden kann (Beispiel 1 und 4). Dabei kann die Inaktivierung vor oder während des proteolytischen Abbaus erfolgen.
Enzyme, die reversibel inaktivierbar sind und deren inaktivierte Form eine erhöhte Empfindlichkeit gegen Proteinasen aufweist, werden erfindungsgemäß Sensor-Enzyme genannt.

Gegenstand der Erfindung ist ein Verfahren zur Bestimmung von Proteinasen mit Hilfe eines Enzyms als Proteinasesubstrat, das dadurch gekennzeichnet ist, daß man ein Sensor-Enzym inaktiviert, mit der zu untersuchenden Probe inkubiert, anschließend das Sensor-Enzym reaktiviert und dessen Restaktivität ermittelt.

Ein weiterer Gegenstand der Erfindung sind Mittel zur Bestimmung von Proteinasen, enthaltend ein Sensor-Enzym als Proteinasesubstrat, eine Inaktivierungslösung und eine Reaktivierungslösung.

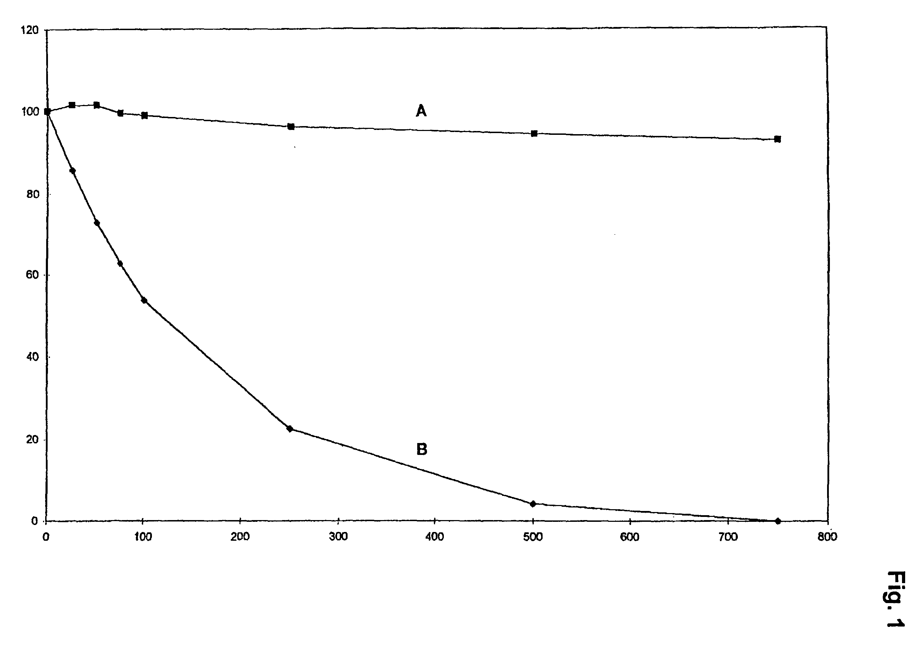

In den Abbildungen 1-4 ist jeweils die Restaktivität [%] eines Substratenzyms (Ordinate) gegen die Proteinasekonzentration (Abszisse) aufgetragen. Nähere Erläuterungen finden sich in den Beispielen 1-4. Die in den Beispielen 1,2 und 4 verwendete Alcalase® ist ein Handelsprodukt der Firma Nycomed Danmark A/S.

Der Vorteil des erfindungsgemäßen Verfahrens liegt in der höheren Nachweisempfindlichkeit, die bei der Verwendung eines Sensor-Enzyms erreicht wird.

Ein weiterer Vorteil des erfindungsgemäßen Verfahrens gegenüber den Methoden, die (modifizierte) Proteine als Substrat verwenden, ist darin zu sehen, daß es z.B. nicht notwendig ist, die Proteolyseprodukte vom Substratprotein zu trennen. Außerdem wird erfindungsgemäß der Testansatz nach der Inkubation verdünnt, bevor die Restaktivität gemessen wird, wodurch Störungen durch Probenbestandteile, wie z.B. Trübungen und Färbungen, verringert werden. Durch die kinetische Messung des Enzyms kann der Einfluß der Farbe bzw. die Trübung der Probe weiter reduziert werden. Dadurch ist es möglich, Proteinasen in Proben wie z.B. Milch zu messen, die mit anderen Methoden nicht oder nicht empfindlich genug gemessen werden können.

Eine wesentliche Eigenschaft von geeigneten Sensor-Enzymen im Sinne der Erfindung ist die reversible Inaktivierung. Sensor-Enzyme müssen unter Bedingungen reversibel inaktiv vorliegen können, unter denen die nachzuweisenden Proteinasen aktiv sind. Anschließend muß das Sensor-Enzym reaktiviert werden können. Enzyme mit diesen Eigenschaften sind aus der Literatur bekannt.
Zu den bekannten Maßnahmen für die reversible Enzymaktivierung gehören Änderungen des Ionenmilieus (Ionenstärke oder pH-Wert), Entfernung der prosthetischen Gruppe oder Metallionen oder die Behandlung mit chaotropen Substanzen. Beispiele für solche Enzyme sind Dehydrogenasen wie Glucose-Dehydrogenasen aus *Bacillus megaterium* oder *Bacillus subtilis* oder Enzyme, die in bekannter Weise durch Entfernung der prosthetischen Gruppe in das inaktive Apoenzym und durch deren Zugabe wieder in das Holoenzym reaktiviert werden können; dazu gehören aus der Klasse der Oxidoreduktasen Glucose-Oxidase, Pyruvat-Oxidase, D-Aminosäure-Oxidase, Xanthin-Oxidase, Lipoamid-Dehydrogenase usw.; Oxidoreduktasen, die Metallionen enthalten, wie Diamin-Oxidase, Ascorbat-Oxidase, Superoxid-Dismutase, Alkohol-Dehydrogenase usw.; Lyasen wie Carboanhydrase; Hydrolasen wie Phosphorylase; Aldolasen wie Alkalische Phosphatase; Transferasen wie Serin-Hydroxymethyltransferase. Weitere geeignete Enzyme für das erfindungsgemäße Verfahren können z.B. solche sein, die durch Harnstoffkonzentrationen unter 2 mol/l inaktiviert und durch Verdünnen des Harnstoffgehalts wieder reaktiviert werden können, wie Alkohol-Dehydrogenasen.

Aus den oben genannten inaktivierbaren Enzymen kann der Fachmann für die nachzuweisende Proteinase geeignete Sensorenzyme auswählen und die Maßnahmen der Inaktivierung und Reaktivierung in geeigneter Weise festlegen.

Die Inaktivierung eines Sensor-Enzyms als Proteinasesubstrat kann z.B. durch Änderung des pH-Wertes und/oder der Ionenstärke, der Temperatur, durch Entfernung einer Effektorsubstanz (z.B. einer prosthetischen Gruppe oder eines Metallions) oder Zugabe einer inaktivierenden Substanz, chemische und physikalische Änderungen der Aminosäuren (z.B. durch Oxidation eines Cysteins) in bekannter Weise erreicht werden. Die Inaktivierung des Sensor-Enzyms kann vor dem Kontakt mit der Probe oder während des Kontaktes erfolgen. Während der Inkubation wird das Enzym inaktiviert und gleichzeitig von der Proteinase gespalten und damit irreversibel inaktiviert.

Die Reaktivierung des Sensor-Enzyms erfolgt in der Regel durch Umkehrung des Inaktivierungsschritts. Dadurch wird der proteolytische Abbau des Enzyms verlangsamt oder gestoppt. Die Reaktivierung des Enzyms kann beispielsweise durch Änderung des pH-Wertes und/oder der Ionenstärke, durch Zugabe eines Effektormoleküls/-ions oder Wegnahme bzw. Verdünnung einer inaktivierenden Substanz erfolgen.

Die Zugabe von Metallionen erfolgt bevorzugt als Metall-Chelatkomplex.

Die Inaktivierung einer Glucose-Dehydrogenase aus *Bacillus megaterium* erfolgt z.B. durch Verschiebung des pH-Wertes in den alkalischen Bereich, vorzugsweise in den pH-Bereich von über pH 7, vorzugsweise pH 8 bis 9, insbesondere wird ein pH-Wert von 8,5 eingestellt. Dabei ist es wichtig, die Ionenstärke des Sensor-Enzyms auf weniger als 500 mmol/l, vorzugsweise auf weniger als 100 mmol/l zu reduzieren, sofern das Enzym in einer hohen Ionenstärke aufbewahrt worden ist. Die Einstellung des gewünschten pH-Wertes wird mit den in der Enzymatik üblichen Pufferlösungen vorgenommen, die einen alkalischen pH-Bereich aufrechterhalten können, wie TRIS/HCI-Puffer, Triethanolamin-Puffer, Veronal-Puffer, Glycylglycin-Puffer, (Trishydroxymethyl)methyl)glycin(TRICINE)-Puffer.

Nach der Mischung der Sensor-Enzymlösung mit dem Inaktivierungspuffer wird etwa 0,5 bis 2 Stunden bei 20 ° bis 37 °C inkubiert. Anschließend wird eine Reaktivierungslösung hinzugegeben, die im Falle von Glucose-Dehydrogenase aus einer Pufferlösung besteht, die einen sauren pH-Bereich von unter 7, vorzugsweise von 5 bis 7, insbesondere einen pH-Wert von etwa 6,0 aufrechterhalten kann. Anstelle einer Pufferlösung kann auch eine Lösung hoher Ionenstärke verwendet werden, z.B. eine Lösung mit einer Kochsalzkonzentration von über 100 mmol/l, vorzugsweise von über 1000 mmol/l. Als Reaktivierungslösung kann auch eine Kombination von Pufferlösung und Lösung hoher Ionenstärke eingesetzt werden. Geeignete Puffer für die saure Reaktivierungslösung sind z.B. Phosphatpuffer, Citratpuffer, Acetatpuffer, 2-Morpholinethansulfonsäure(MES)-Puffer vorzugsweise Phosphatpuffer in einem Konzentrationsbereich von 50 bis 500 mmol/l.

Nach der Zugabe der Reaktivierungslösung wird etwa 0,2 bis 0,5 Stunden bei 20 ° bis 37 °C inkubiert. Anschließend wird die Glucose-Dehydrogenase-Aktivität nach bekannten Methoden bestimmt und daraus die Proteinaseaktivität ermittelt.

Die Bestimmung von Proteinasen mit Glucose-Oxidase als Substrat wird in bekannter Weise so durchgeführt, daß mit Hilfe einer sauren Inaktivierungslösung (z.B. pH 1,5) die prosthetische Gruppe FAD entfernt wird. Die Reaktivierung nach der Inkubation erfolgt durch Zugabe von FAD.

Die Proteinasebestimmungen erfolgen in der Weise, daß jede Meßreihe einen Leerwert (Wasser bzw. Probe ohne Proteinase) enthält, auf den nach Inkubation und Reaktivierung die Restaktivität des Sensor-Enzyms auf 100% bezogen wird. Proben mit proteolytischer Aktivität ergeben eine geringere Restaktivität. Mit definierten Mengen einer Proteinase kann eine Standardkurve erstellt und die Aktivität einer unbekannten Probe kann aus dieser Standardkurve abgelesen werden.

Auch ohne weitere Ausführungen wird davon ausgegangen, daß ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

Die vollständige Offenbarung aller vor- und nachstehend aufgeführten Anmeldungen, Patente und Veröffentlichungen, sowie der korrespondierenden Anmeldung DE 19814761, eingereicht am 02.04.1998, sind durch Bezugnahme in diese Anmeldung eingeführt.

### Beispiel 1 Bestimmung der proteolytischen Aktivität von Alcalase® in Pufferlösung mit Glucose-Dehydrogenase

Zu je 1 ml einer Inaktivierungslösung aus 50 mmol/l TRIS/HCI-Puffer, pH 8,5 und 0,2 mg/ml Rinderserumalbumin werden 100 µl Alcalase®verdünnungen, enthaltend 0, 25, 50, 75, 100, 250, 500, 750 ng/ml Alcalase® (0,6 Anson-Einheiten pro g) in TRIS/HCI-Puffer, gegeben. Nach der Zugabe von je 50 µl Glucose-Dehydrogenase aus *Bacillus megaterium* (0,2 mg/ml in 13 mmol/l HEPES-Puffer, 0,4 mol/l Natriumchlorid, pH 6,8) wird bei 37 °C 2 Stunden inkubiert. Danach werden jeweils 1 ml Reaktivierungslösung (200 mmol/l Natriumphosphatpuffer, pH 6 und 2 mol/l Natriumchlorid) hinzugegeben und weitere 30 Minuten bei 37 °C inkubiert.

Die Messung der Restaktivität erfolgt photometrisch in Halbmikroliterküvetten bei 340 nm und 25 °C. Dazu werden 1 ml Substratlösung (0,2 mol/l Phosphatpuffer, pH 7,2; 1 % D-Glucose) und 0,1 ml NAD-Lösung (0,1 mol/l) in die Küvetten pipettiert. Der Start der Messung erfolgt durch Zugabe von je 50 µl des Inkubationsansatzes.

Zur Auswertung wird die Verdünnungskurve erstellt, wobei aus ΔE Probe und ΔE Leerwert die Restaktivität [%]des Substratenzyms berechnet und gegen die Konzentration der Proteinase (Alcalase®) [ng/ml] aufgetragen wird (Fig. 1, Kurve B). Der Leerwert (Probe ohne Alcalase®) entspricht 100 % Restaktivität; die Restaktivitäten (%) der Alcalase®proben berechnen sich aus ΔE (Probe) x 100/ΔE (Leerwert).

Zum Vergleich wird die Bestimmung unter Bedingungen durchgeführt, bei denen die Glucose-Dehydrogenase aktiv bleibt (Zusatz von 0,5 mol/l Natriumchlorid). Das Ergebnis zeigt Fig. 1, Kurve A. Wie aus Fig. 1 zu entnehmen ist, ist die Glucose-Dehydrogenase in Kurve B mindestens um den Faktor 30 sensitiver gegenüber der Alcalase® als in Kurve A, d.h., daß das Verfahren zu Bestimmung der Alcalase® mehr als 30mal empfindlicher ist.

### Beispiel 2 Bestimmung der Aktivität von Alcalase® in Milch mit Glucose-Dehydrogenase

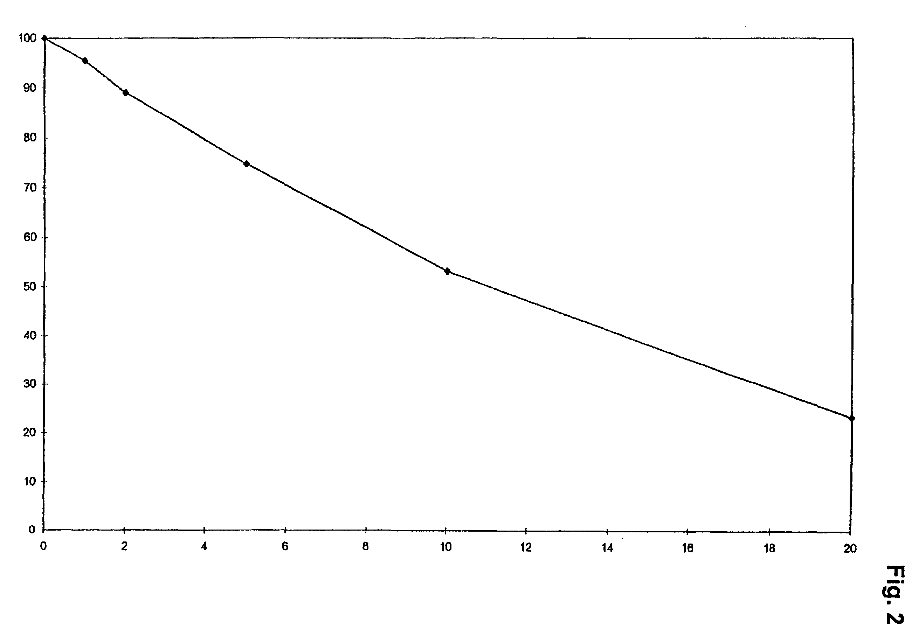

Die Bestimmung wird analog Beispiel 1 durchgeführt, wobei anstelle von TRIS/HCI-Puffer pasteurisierte Milch zum Verdünnen der Alcalase® verwendet wird. Die Alcalase®konzentration in Milch beträgt 0, 1, 2, 5, 10 und 20 µg/ml. Das Ergebnis zeigt Fig. 2: Proportional zur Konzentration der Alcalase® [µg/ml] sinkt die Restaktivität [%] der Glucose-Dehydrogenase. Damit ist eine Bestimmung der Alcalase®konzentration auch in Milch mit der beschriebenen Methode möglich.

### Beispiel 3 Bestimmung der Trypsinaktivität in Pufferlösung mit Glucose-Dehydrogenase

Zu je 1 ml einer Inaktivierungslösung (50 mmol/l TRIS/HCI-Puffer, pH 8,5 und 0,2 mg/ml Rinderserumalbumin) werden 100 µl der Trypsinverdünnungen enthaltend 0, 5, 10, 20, 50, 100 und 200 ng/ml zu der oben genannten Inaktivierungslösung gegeben. Nach der Zugabe von je 50 µl Glucose-Dehydrogenase aus *Bacillus megaterium* (0,2 mg/ml) wird bei 37 °C 2 Stunden lang inkubiert. Danach wird jeweils 1 ml Reaktivierungslösung (200 mmol/l Natriumphosphatpuffer, pH 6, 2 mol/l Natriumchlorid) hinzugegeben und weitere 30 Minuten bei 37 °C inkubiert.

Die Messung der Restaktivität erfolgt photometrisch in Halbmikroliterküvetten bei 340 nm und 25 °C. Dazu werden 1 ml Substratlösung (0,2 mol/l Phosphatpuffer, pH 7,2; 1 % D-Glucose) und 0,1 ml NAD-Lösung (0,1 mol/l) in die Küvetten pipettiert. Der Start der Messung erfolgt durch Zugabe von je 50 µl des Inkubationsansatzes.

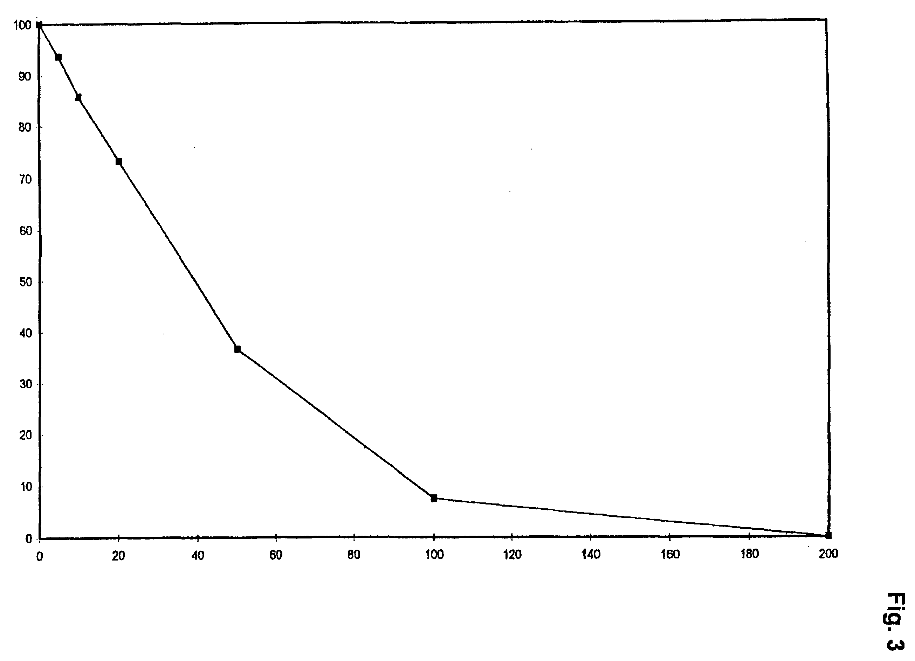

In Fig. 3 ist die Restaktivität [%] an Glucose-Dehydrogenase gegen die Trypsinkonzentration [ng/ml] aufgetragen. Die Kurve zeigt proportional zur Konzentrationszunahme von Trypsin eine Abnahme der Restaktivität der Glucose-Dehydrogenase. Eine Trypsinkonzentration in der Probe von 5 ng/ml (entspricht 0,43 ng/ml im Testansatz) ergibt eine Restaktivität von 93,6 % und eine Trypsinkonzentration von 10 ng/ml (entspricht 0,87 ng/ml im Testansatz) resultiert in einer Restaktivität von 85,7 %. Dies zeigt, daß es möglich ist, Proteasen auch mit einer Konzentration von unter 1 ng/ml im Testansatz bestimmen zu können.

### Beispiel 4 Bestimmung der Aktivität von Alcalase® mit Glucose-Oxidase

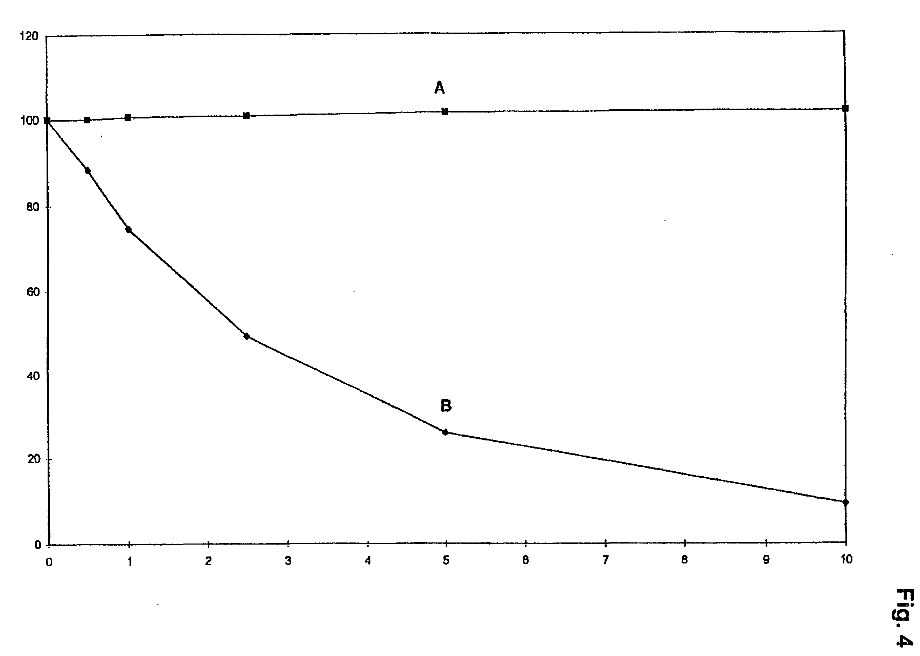

Glucose-Oxidase aus *Aspergillus niger* wird bei saurem pH-Wert inkubiert und dabei durch Entfernung der prosthetischen Gruppe FAD zur inaktiven Apo-Glucose-Oxidase umgewandelt:
0,934 g Glucose-Oxidase werden in 20 ml 0,1 mol/l Glycin/HCl Puffer, pH 1,5 und 30 % w/v Glycerin im Eisbad 1 Stunde gerührt. Die Lösung wird über eine Gelfiltrationssäule gegeben, um das Apoenzym von FAD abzutrennen. Das Apoenzym wird nach der Chromatographie mit TRIS neutralisiert und bei 4 °C aufbewahrt.
Zu je 50 µl einer Inkubationslösung (25 mmol/l TRIS/HCI, pH 7,6) mit Glucose-Oxidase (10 µg/ml) und Apo-Glucose-Oxidase (100 µg/ml) in einer Mikrotiterplatte werden 10 µl Alcalase®verdünnung enthaltend 0, 0,5, 1, 2,5, 5 und 10 µg/ml gegeben. Nach Mischen wird die Mikrotiterplatte bei Raumtemperatur für 30 Minuten inkubiert. Danach wird zur Reaktivierung der Apo-Glucose-Oxidase 50 µl FAD (10 µg/ml) bzw. 50 µl Wasser zur Glucose-Oxidase und jeweils 100 µl Citrat/Phosphatpuffer pH 5,5 (8,88 g Citronensäure und 20,57 g Natriumhydrogenphosphat in 1 l Wasser) gegeben und 10 Minuten bei Raumtemperatur inkubiert. Die Aktivität der Glucose-Oxidase wird nach Zugabe von 50 µl Substratlösung (0,375 g Tetramethylbenzidin in 5 ml Dimethylsulfoxid, Methanol ad 25 ml, davon 50 µl in 10 ml Citrat/Phosphatpuffer pH 5,5, 130 mg Glucose, 2 µl Peroxidase: 2,6 mg/ml = 650 U/ml) und Inkubation bei Raumtemperatur nach 5-10 Minuten bestimmt. Nach Zugabe von 50 µl Schwefelsäure 5 % wird bei 450 nm in einem Photometer zur Messung von Proben in Mikrotiterplatten gemessen.
Zur Auswertung wird die Verdünnungskurve erstellt, wobei aus ΔE Probe und ΔE Leerwert die Restaktivität [%] des Substratenzyms berechnet und gegen die Konzentration [µg/ml] der Proteinase (Alcalase®) aufgetragen wird (Fig. 4). Der Leerwert (Probe ohne Alcalase®) entspricht 100 % Restaktivität; die Restaktivitäten (%) der Alcalase®proben berechnen sich aus ΔE (Probe) x 100/ΔE (Leerwert). Zum Vergleich wird die Bestimmung mit aktiver Glucose-Oxidase durchgeführt. Wie aus Fig. 4 zu entnehmen ist, ist die Apo-Glucose-Oxidase in Kurve B mindestens um den Faktor 20 sensitiver gegenüber proteolytischem Abbau durch Alcalase® als die Glucose-Oxidase in Kurve A, d.h., daß das Verfahren zur Bestimmung der Alcalase® mehr als 20mal empfindlicher ist.

## Patentansprüche

1. Verfahren zur Bestimmung von Proteinasen mit Hilfe eines Enzyms als Proteinasesubstrat, **dadurch gekennzeichnet, daß** man ein Sensor-Enzym inaktiviert, mit der zu untersuchenden Probe inkubiert, anschließend das Sensor-Enzym reaktiviert und dessen Restaktivität ermittelt.

2. Verfahren nach Anspruch 1, **dadurch gekennzeichnet, daß** als Sensor-Enzyme Oxidoreduktasen verwendet werden.

3. Verfahren nach einem der Ansprüche 1 oder 2, **dadurch gekennzeichnet, daß** die Inaktivierung und Reaktivierung des Sensor-Enzyms durch Änderung des pH-Wertes und/oder der Ionenstärke erfolgt.

4. Verfahren nach einem der Ansprüche 1 oder 2, **dadurch gekennzeichnet, daß** die Inaktivierung und Reaktivierung des Sensor-Enzyms durch Entfernung und Zugabe eines Effektormoleküls bzw. durch Zugabe und Entfernung einer inaktivierenden Substanz erfolgt.

5. Mittel zur Bestimmung von Proteinasen, umfassend ein Sensor-Enzym, eine Inaktivierungslösung und eine Reaktivierungslösung für das Sensor-Enzym.

6. Mittel nach Anspruch 5, umfassend Glucose-Dehydrogenase als Sensor-Enzym, eine Inaktivierungslösung von schwach alkalischem pH-Wert und eine Reaktivierungslösung von schwach saurem pH-Wert.

7. Mittel nach Anspruch 5, umfassend Apo-Glucose-Oxidase als Sensor-Enzym und eine FAD-enthaltende Reaktivierungslösung.

## Claims

1. Process for the determination of proteinases with the aid of an enzyme as proteinase substrate, **characterised in that** a sensor enzyme is deactivated, incubated with the sample to be investigated, the sensor enzyme is subsequently reactivated and its residual activity is determined.

2. Process according to Claim 1, **characterised in that** the sensor enzymes used are oxidoreductases.

3. Process according to one of Claims 1 or 2, **characterised in that** the deactivation and reactivation of the sensor enzyme is carried out by changing the pH and/or the ion strength.

4. Process according to one of Claims 1 or 2, **characterised in that** the deactivation and reactivation of the sensor enzyme is carried out by removal and addition of an effector molecule or by addition and removal of a deactivating substance.

5. Composition for the determination of proteinases comprising a sensor enzyme, a deactivation solution and a reactivation solution for the sensor enzyme.

6. Composition according to Claim 5, comprising glucose dehydrogenase as sensor enzyme, a deactivation solution of weakly alkaline pH and a reactivation solution of weakly acidic pH.

7. Composition according to Claim 5, comprising apo-glucose oxidase as sensor enzyme and an FAD-containing reactivation solution.

## Revendications

1. Procédé pour la détermination de protéinases à l'aide d'une enzyme en tant que substrat de protéinase, **caractérisé en ce qu'**une enzyme de capteur est désactivée et incubée avec l'échantillon à investiguer, l'enzyme de capteur est ensuite réactivée et son activité résiduelle est déterminée.

2. Procédé selon la revendication 1, **caractérisé en ce que** les enzymes de capteur utilisées sont des oxydoréductases.

3. Procédé selon l'une des revendications 1 ou 2, **caractérisé en ce que** la désactivation et la réactivation de l'enzyme de capteur sont mises en oeuvre en modifiant le pH et/ou l'intensité ionique.

4. Procédé selon l'une des revendications 1 ou 2, **caractérisé en ce que** la désactivation et la réactivation de l'enzyme de capteur sont mises en oeuvre par enlèvement et addition d'une molécule effectrice ou par addition et enlèvement d'une substance de désactivation.

5. Composition pour la détermination de protéinases comprenant un enzyme de capteur, une solution de désactivation et une solution de réactivation pour l'enzyme de capteur.

6. Composition selon la revendication 5, comprenant une déhydrogénase de glucose en tant qu'enzyme de capteur, une solution de désactivation de pH faiblement basique et une solution de réactivation de pH faiblement acide.

7. Composition selon la revendication 5, comprenant une oxydase apo-glucose en tant qu'enzyme de capteur et une solution de réactivation contenant FAD.
